Europäisches Patentamt

European Patent Office

Office européen des brevets

(11) Publication number: 0 648 781 A1

# EUROPEAN PATENT APPLICATION
## published in accordance with Art. 158(3) EPC

(21) Application number: 93913543.0

(22) Date of filing: 18.06.93

(86) International application number: PCT/JP93/00823

(87) International publication number: WO 94/00490 (06.01.94 94/02)

(51) Int. Cl.⁶: $C07K\ 11/02$, $C07K\ 1/14$, $A61K\ 37/02$

(30) Priority: 23.06.92 JP 187448/92

(43) Date of publication of application:
19.04.95 Bulletin 95/16

(84) Designated Contracting States:
DE FR GB IT

(71) Applicant: NIPPON KAYAKU KABUSHIKI KAISHA
11-2, Fujimi 1-chome
Chiyoda-ku
Tokyo 102 (JP)

(72) Inventor: OHKUMA, Takaaki
5-13, Fujimi 2-chome,
Fukiagemachi

Kitaadachi-gun,
Saitama 369-01 (JP)
Inventor: HIRAGA, Hironobu
46, Honmachi 8-chome
Kitamoto-shi,
Saitama 364 (JP)
Inventor: IGARASHI, Hisae
3-26-21, Takanodai
Nerima-ku,
Tokyo 177 (JP)

(74) Representative: Türk, Dietmar, Dr. rer. nat.
Türk, Gille, Hrabal, Leifert
Patentanwälte
Brucknerstrasse 20
D-40593 Düsseldorf (DE)

(54) NONHYGROSCOPIC CRYSTAL OF AUREOBASIDIN A AND PRODUCTION THEREOF.

(57) Nonhygroscopic crystal of aureobasidin A, and a process for producing nonhygroscopic aureobasidin A by dissolving noncrystalline aureobasidin A in a neutral fat or a polyethylene glycol, followed by deposition therefrom. The obtained crystal is non-hygroscopic unlike the conventional noncrystalline powder and is suited for preparing pharmaceuticals.

FIG. I

EP 0 648 781 A1

The present invention relates to a non-hygroscopic crystal of aureobasidin A which are useful as an antifungal agent, and a process for production thereof.

## BACKGROUND ART

Aureobasidin A (identical with the novel antibiotic R106-I; hereinafter referred to as R106-I) is an antibiotic produced by a strain belonging to Genus Aureobasidium (international deposit number: FERM-BP1938), and its usefulness as an antifungal agent is expected (JP-A-2-138296, JP-A-3-22995). R106-I is a cyclic peptide having a molecular weight of 1,101. Up to today, this substance has been available as an amorphous white-colored powder.

This amorphous white-colored powder is hygroscopic. Generally speaking, the content of active ingredient in a pharmaceutical preparation must be kept constant. When the active ingredient is hygroscopic, the process for production of pharmaceutical preparation would be difficult to control because the water content of the active ingredient must be kept always constant and the like. For this reason, active ingredient is required to be non-hygroscopic. However, R106-I is a cyclic polypeptide difficult to crystallize, and it is available only as an amorphous hygroscopic powder.

## DISCLOSURE OF INVENTION

In the course of studying the physico-chemical properties of R106-I, the present inventors have found that naturally occurring neutral fatty oils (vegetable oils), synthetic and semi-synthetic neutral fatty oils or polyethylene glycols which is a liquid state at ordinary temperature are, even though these substances are not used as a solvent in the conventional crystallization processes, as good solvents for the amorphous R106-I, and that a crystal prepared by dissolving R106-I in these solvents followed by deposition of crystal is non-hygroscopic unlike the so far known amorphous powder and has a good purity. Based on these findings, the present invention has been accomplished.

Thus, the first aspect of the present invention relates to a non-hygroscopic crystal of R106-I.

This crystalline powder exhibits an endothermic peak due to a rapid melting usually between about 200°C and about 206°C as determined by differential thermal analysis, though the peak temperature may somewhat vary depending on purity.

The second aspect of the present invention relates to a process for producing the above-mentioned non-hygroscopic crystal of R106-I. This process of production is characterized by dissolving R106-I in a neutral fatty oil or polyethylene glycol and depositing a crystal of R106-I from the solution.

## BRIEF DESCRIPTION OF THE DRAWINGS

In Fig. 1, (a), (b) and (c) illustrate endothermic change curves determined by differential scanning calorimetry (DSC curves) of (a) a hitherto known amorphous sample, (b) the crystalline powder obtained in Example 3 and (c) the crystalline powder obtained in Example 2, respectively.

In Fig. 2, (A), (B) and (C) illustrate X-ray diffraction patterns of (A) a hitherto known amorphous sample, (B) the crystal obtained in Example 3 and (C) the crystalline powder obtained in Example 2, respectively.

## BEST MODE FOR CARRYING OUT THE INVENTION

Hereinunder, the present invention is explained concretely.

The neutral fatty oils used in the method of this invention as a solvent include naturally occurring vegetable oils and synthetic and semi-synthetic neutral fatty oils. These neutral fatty oils are preferably in a liquid state at the temperature at which a crudely purified product of R106-I is dissolved and crystallized.

As the naturally occurring neutral fatty oil, corn oil, soybean oil, sesame oil, coconut oil, olive oil, peanut oil, castor oil and the like can be used. As the synthetic and semi-synthetic neutral fatty oils, triglycerides of medium chain ($C_6$ to $C_{12}$) fatty acids, such as caprylic acid triglyceride, capric acid triglyceride and the like, can be used.

When these solvents are used, crystallization can be practiced in the following manner. Thus, a crudely purified product of amorphous R106-I is dissolved in a liquid neutral fatty oil with, for example, stirring and then the solution is left to stand, whereby a crystal of R106-I is formed in the solvent. Although it is usually desirable to dissolve R106-I in the neutral fatty oil at ordinary temperature, namely in the temperature range of from about 10°C to about 35°C, the dissolution may be carried out under heating or under cooling, when occasion demands.

The quantity of dissolution of R106-I in a neutral fatty oil may vary depending on the kind of neutral fatty oil used or the temperature of dissolution, etc., it is usually preferable to dissolve R106-I until it reaches a saturation or a supersaturation.

Generally speaking, it is desirable to select the solvent and conditions of dissolution so that from about 1% W/W to about 30% W/W, preferably 5% W/W or more, of R106-I becomes dissolved based on neutral fatty oil.

For depositing the crystal from the solvent, a solution in which R106-I is dissolved may be allowed to stand as it is. Otherwise, when the dissolution has been attained at ordinary temperature, the resulting solution may be heated to a temperature of from about 30°C to about 70°C and maintained at that temperature as it is in order to accelerate the deposition of crystal. Addition of successive small portions of a hydrocarbon type organic solvent functioning as a poor solubility of R106-I and a good solubility of neutral fatty oil, such as those having about 6-10 carbon atoms (for example, hexane and the like), is also effective for accelerating the crystallization.

The crystal thus formed is collected by filtration, washed with a solvent functioning as a poor solubility of R106-I and a good solubility of neutral fatty oil, and then dried, whereby the objective non-hygroscopic crystal is obtained. This crystal exhibits no substantial hygroscopic character.

The polyethylene glycols which can be used as a solvent similarly to the neutral fatty oils in the process of the present invention include those presenting a liquid state at the working temperature, such as Polyethylene Glycol 300, Polyethylene Glycol 400, Polyethylene Glycol 600 and the like. The purification by crystallization using these solvents can also be practiced in the following manner, similarly to the case of using a neutral fatty oil.

Thus, an amorphous powder of R106-I is dissolved in polyethylene glycol with, for example, stirring and thereafter left to stand, whereby a crystal of R106-I is formed in the solvent.

The dissolution of R106-I in polyethylene glycol may be practiced at ordinary temperature usually, though it may also be carried out under heating or under cooling, when occasion demands. Although the quantity of R106-I to be dissolved in polyethylene glycol cannot simply be decided because it depends on the kind of polyethylene glycol and the temperature of dissolution, it is usually from about 3% W/W to about 30% W/W.

For deposition of R106-I crystal from the polyethylene glycol solution, the solution is left to stand as it is. When the formation of crystal is slow, a solvent which is a poor solubility of R106-I and readily soluble in polyethylene glycol, such as water, may be added in successive small portions, which effectively accelerates the crystallization.

The crystal thus formed is collected by filtration, washed with a solvent which is a poor solubility of R106-I and a good solubility of polyethylene glycol, and then dried, whereby a non-hygroscopic crystal of R106-I can be obtained. This crystal exhibits no substantial hygroscopic character.

The R106-I to be crystallized according to the process of the present invention is usually an amorphous powder which has been purified over 80-90% or more. When occasion demands, however, an amorphous powder of lower purity, for example those having a purity of about 50%, are also usable.

The amorphous powder of R106-I can be produced according to, for example, the method disclosed in JP-A-2-138296 (USA-5057493, USA-5158876, EPA-352092). The descriptions of these papers are incorporated by references.

WORKING EXAMPLES

Hereinunder, examples of the present invention are presented to explain the invention more concretely. The present invention is by no means limited by these examples.

Example 1

A clear solution was prepared by dissolving 500 mg of a crudely purified product of amorphous R106-I containing 5.2% of impurities, obtained according to the column purification method (cf. JP-A-2-138296, USA-5057493, USA-5158876, EPA-352092), in 10 g of corn oil. The solution was stirred at 50°C, and a turbidity became noticeable in several hours due to formation of a crystal. Subsequently, the solution was allowed to stand for 24 hours at 50°C, and there was formed a precipitate of the crystal at the bottom of the vessel. The precipitate was collected by filtration by a glass filter and washed with 15 ml of n-hexane. After air-dryness on the glass filter, the precipitate was dried in vacuum at room temperature to obtain a white-colored crystalline powder. Yield 398 mg (79.6%); purity 96%; impurity content 4%; mp. 200.6°C

Example 2

A clear solution was prepared by dissolving 5.0 g of an amorphous powder of R106-I containing 3.6% of impurities, obtained according to the column purification method, in 25 g of caprylic acid triglyceride (Panacet 800, manufactured by NIPPON OIL & FATS CO., LTD). The solution was stirred at 50°C, and a turbidity became noticeable in several hours due to formation of a crystal. Subsequently, the solution was allowed to stand for 24 hours at 50°C, and a crystal was deposited in the whole solution. The crystal was collected by filtration by a glass filter and washed with 50 ml of n-hexane. After air-dryness on the glass filter, the crystalline matter was dried in vacuum at room temperature to obtain a white-colored crystalline powder. Yield 3.78 g (75.6%); purity 97.4%; impurity content 2.6%; mp. 202.2°C

Example 3

A clear solution was prepared by dissolving 500 mg of an amorphous powder of R106-I containing 5.2% of impurities, obtained according to the column purification method, in 2 g of Polyethylene Glycol 400. The solution was stirred at 50°C, and a turbidity became noticeable in several hours due to formation of a crystal. Subsequently, the solution was allowed to stand for 24 hours at 50°C, and a crystal was deposited in the whole solution. The crystal was collected by filtration by a glass filter and washed with 15 ml of purified water. After air-dryness on the glass filter, the crystalline matter was dried in vacuum at room temperature to obtain a white-colored crystalline powder. Yield 0.442 g (88.4%); purity 95.5.%; mp. 197.8°C

Test Example 1: Comparison of hygroscopic property of prior amorphous powder with that of crystalline powder of the present invention

(1) Samples

Amorphous sample:
An amorphous powder of R106-I prepared by the same procedure as used for preparation of the amorphous sample used in Example 1
Crystalline sample:
A crystalline powder of R106-I prepared in the same manner as in Example 2

(2) Moisture-absorbing test

1) Drying before test: The amorphous and crystalline powdery samples were dried at 100°C for 4 hours
2) Conditions of moisture absorption: Temperature 25°C, relative humidity 93% (saturated $KNO_3$ solution)
3) Procedure of experiment: The amorphous powdery sample and the crystalline powdery sample were accurately weighed into weighing bottles, subjected to a drying before test, and stored in a desiccator kept under the above-mentioned conditions, while periodically measuring the weights of the samples. After confirming that the increases in weights due to moisture absorption reached equilibrium, equilibrium moisture content were calculated.
4) Calculation: Equilibrium moisture content (% by weight) were calculated according to the following equation:

Equilibrium moisture content (% by weight)
= (Sample weight after equilibrium
- Sample weight after dryness before test)
/ (Sample weight after dryness before test)

× 100

(3) Results

Amorphous sample:
Equilibrium moisture content : 3.0% by weight
Crystalline sample:
Equilibrium moisture content : 0.0% by weight
The amorphous sample exhibited a moisture absorption of 3% at a relative humidity of 93% RH, while the crystalline sample exhibited no noticeable moisture absorption at all.

Test Example 2: Differential scanning calorimetric analyses (DSC analyses)

(1) Samples

Sample (a):     Amorphous sample
Sample (b):     Crystalline powder obtained in Example 3, crystallized with Polyethylene Glycol 400
Sample (c):     Crystalline powder obtained in Example 2, crystallized with caprylic acid triglyceride

(2) Conditions of measurement

Apparatus for measurement:
SHIMAZU Thermal Analyzer TA-50, DSC-50
Conditions:
Temperature elevation rate: 10°C/min Temperature range of measurement:
    Room temperature to 350°C
    Sample weight: ca. 5-6 mg

(3) Results

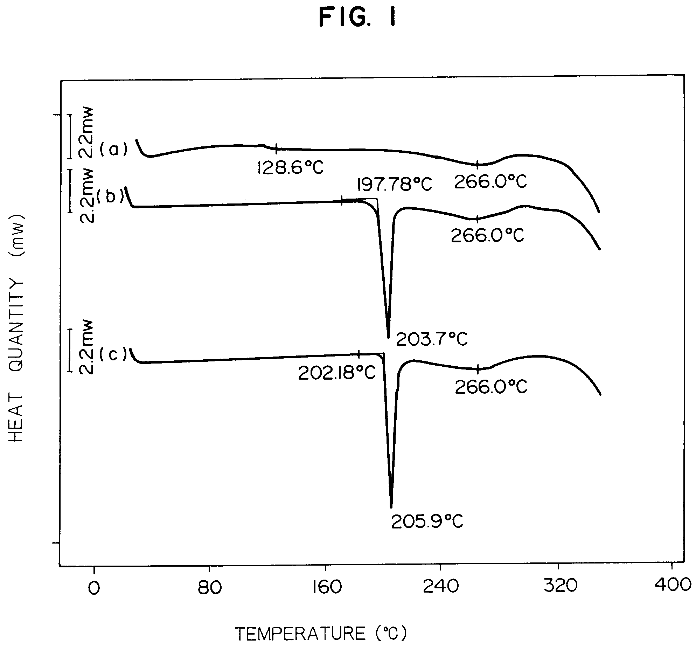

The results are shown in Fig. 1.
The DSC curves are those of Samples (a), (b) and (c) in order from the top. The abscissa expresses temperature (°C), and the ordinate expresses heat quantity (mw).
While the amorphous sample showed no explicit peak, the crystalline samples showed an endothermic peak due to melting of crystal at about 200°C to 206°C. In an observation using a polarizing microscope, the crystalline samples showed a polarization, while the amorphous sample showed no polarization. Based on the polarization shown by the crystal and the endothermic peaks shown in the differential thermal analyses, it could be confirmed that samples (b) and (c) were crystals.
Apart from the above, a sample recrystallized from corn oil also showed a similar DSC curve.

Test Example 3: Crystalline powder X-Ray analyses

(1) Samples

| Sample (a): | Amorphous sample |
| Sample (b): | Crystalline powder obtained in Example 3, crystallized with Polyethylene Glycol 400 |
| Sample (c): | Crystalline powder obtained in Example 2, crystallized with caprylic acid triglyceride |

(2) Conditions of measurement

Apparatus for measurement:
Crystalline Powder X-Ray Diffraction Analyzer RAD-IIC (manufactured by Rigaku Co., Ltd.)
Conditions of measurement:
Generation of X-ray: Copper target, nickel filter, voltage 30 KV, current 10 mA
Detection of X-ray: Scintillation counter, scanning speed 2°/min

(3) Results

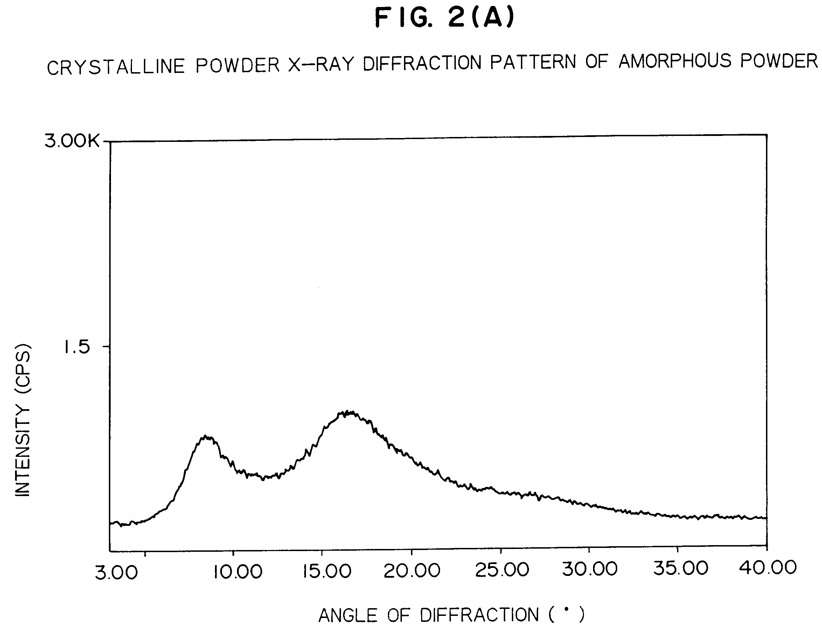

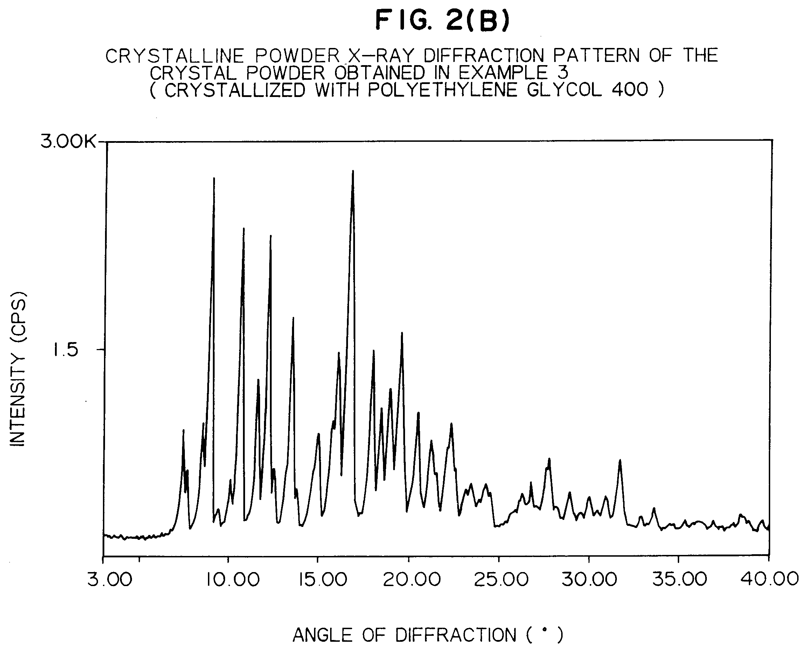

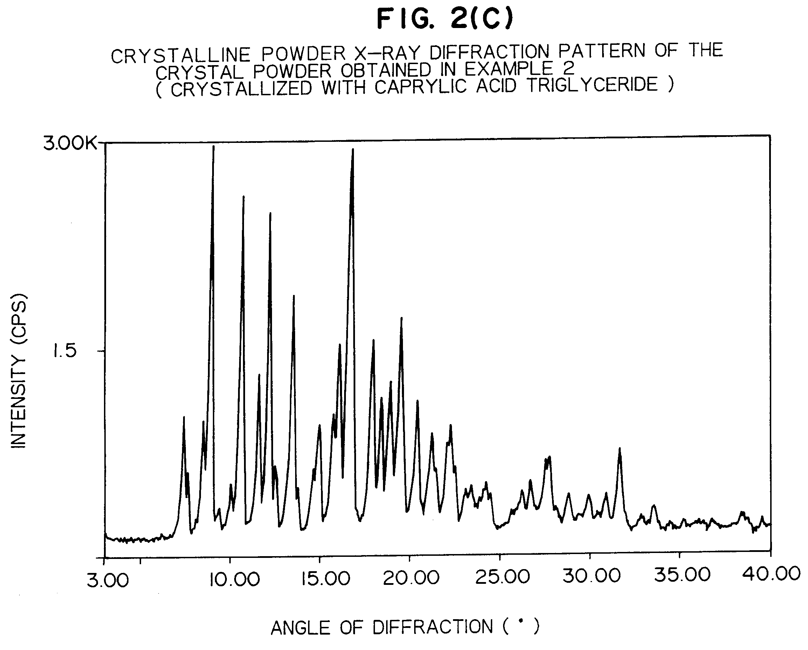

The results are shown in Fig. 2 (A), (B) and (C). In Fig. 2, (A), (B) and (C) each expresses diffraction pattern of Samples (a), (b) and (c), respectively, provided that the abscissa expresses diffraction angle (°) and the ordinate expresses intensity (CPS).

Unlike the diffraction pattern of the amorphous sample (a) (Fig. 2 (A)), the diffraction patterns of the crystalline samples (b) and (c) showed main peaks at the following diffraction angles (Fig. 2 (B) and (C)):
Diffraction angles:
7.4, 8.5, 8.9, 10.6, 11.6, 12.2, 13.5, 16.0, 16.7, 17.9, 18.4, 18.9, 19.5

INDUSTRIAL APPLICABILITY

The non-hygroscopic crystal of R106-I according to the present invention exhibits no hygroscopic character at all, and is suitable for use in manufacture of a preparation. Further, the process of the present invention has enabled crystallization of R106-I for the first time, and reduction of impurity content became noticeable by the crystallization.

**Claims**

1. A non-hygroscopic crystal of aureobasidin A.

2. A non-hygroscopic crystal of aureobasidin A exhibiting an eodothermic peak between about 200°C and about 206°C due to melting and having a diffraction pattern showing main peaks at the following diffraction angles:
7.4°, 8.5°, 8.9°, 10.6°, 11.6°, 12.2°, 13.5°, 16.0°, 16.7°, 17.9°, 18.4°, 18.9° and 19.5°.

3. A process for production of a non-hygroscopic crystal of aureobasidin A characterized by dissolving an amorphous aureobasidin A in a neutral fatty oil or polyethylene glycol, followed by depositing a crystal from the solution.

# FIG. I

EP 0 648 781 A1

# FIG. 2(A)

CRYSTALLINE POWDER X—RAY DIFFRACTION PATTERN OF AMORPHOUS POWDER

ANGLE OF DIFFRACTION ( ° )

EP 0 648 781 A1

# FIG. 2(B)

CRYSTALLINE POWDER X—RAY DIFFRACTION PATTERN OF THE
CRYSTAL POWDER OBTAINED IN EXAMPLE 3
( CRYSTALLIZED WITH POLYETHYLENE GLYCOL 400 )

INTENSITY (CPS)

ANGLE OF DIFFRACTION ( ° )

EP 0 648 781 A1

# FIG. 2(C)

CRYSTALLINE POWDER X—RAY DIFFRACTION PATTERN OF THE
CRYSTAL POWDER OBTAINED IN EXAMPLE 2
( CRYSTALLIZED WITH CAPRYLIC ACID TRIGLYCERIDE )

INTENSITY (CPS)

ANGLE OF DIFFRACTION ( ° )

EP 0 648 781 A1

INTERNATIONAL SEARCH REPORT

International application No.

PCT/JP93/00823

**A. CLASSIFICATION OF SUBJECT MATTER**

Int. $Cl^5$  C07K11/02, 1/14, A61K37/02

According to International Patent Classification (IPC) or to both national classification and IPC

**B. FIELDS SEARCHED**

Minimum documentation searched (classification system followed by classification symbols)

Int. $Cl^5$  C07K11/00, 7/06, 1/00, A61K37/02

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 2-138296 (Takara Shuzo Co., Ltd.), May 28, 1990 (28. 05. 90), Pages 1 to 3 & EP, A, 352092 & US, A, 5057493 | 1-3 |
| P | J. Chem. Soc., Chem. Commun., No. 17 (1992), Toshimasa Ishida et al., "Conformational feature of aureobasidin E, a new type of potent antifungal antibiotic"  pp. 1231-1233 | 1-3 |

☐ Further documents are listed in the continuation of Box C.      ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| September 1, 1993 (01. 09. 93) | September 28, 1993 (28. 09. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)